# EUROPEAN PATENT APPLICATION

(11) **EP 1 780 190 A1**
(43) Date of publication of application: **02.05.2007**
(21) Application number: 05758325.4
(22) Date of filing: 11.07.2005
(51) Int. Cl.: C07C 13/615, C07C 7/00

(54) **METHOD OF PREVENTING ADAMANTANE CONSOLIDATION**

(30) Priority: 15.07.2004 JP 2004208394
(71) Applicant: IDEMITSU KOSAN CO., LTD., Tokyo 100-8321 (JP)
(72) Inventor: MASE, Jun, 2990193 (JP); KUSABA, Toshiaki, 2990193 (JP)
(74) Representative: Vossius & Partner
(86) International application number: PCT/JP2005/012761
(87) International publication number: WO 2006/008998

(57) **Abstract**

The present invention provides a method of controlling the amount of the solvent contained in adamantane purified by using a naphthene-based solvent to 0.35% by mass or less, or controlling the amount of the solvent contained in adamantane purified by using a non-naphthene-based solvent to 0.05% by mass or less and storing adamantane at 35°C or lower. By this method, adamantane whose particles do not agglomerate together or cake and having stable quality can be obtained.

## Description

### Technical Field

The present invention relates to a method of preventing adamantane from caking, more specifically to a method of preventing adamantane from caking during storage or transportation.

### Background Art

Adamantane is generally used as an industrial chemical product, in particular as a raw material of chemical products, medical drugs, monomeric base materials for photoresists and the like, and the demand for this substance recently increases. Moreover, adamantane derivatives, which are fabricated from adamantane, are also expected to be useful for similar applications.
Such adamantanes are synthesized by using dicyclopentadiene as a raw material and a Lewis acid as a catalyst, such as aluminium chloride. They are normally subjected to a washing step with an organic solvent, and then shipped and marketed as industrial chemical products packed in drums or paper bags.

The melting point of adamantane is as high as 268°C, but it has a high vapor pressure at room temperature and sublimes. Therefore, adamantane is characteristically likely to cake during storage and transportation. Once adamantane is caked, agglomeration proceeds strongly until it maintains the shape of the package. This creates the necessity for fabricating manufacturers to pulverize the agglomeration when taken out from the container. Such extremely poor operability has been a big problem among persons of skill in the art. Although such a problem has been recognized, adamantane has been marketed in a form containing caking without effective measures.
In such a situation, as a method for obtaining adamantane with little caking, granulation of adamantane powder by roller compaction or slug tableting to inhibit the growth of sublimate crystals and prevent concretion has been disclosed (see Patent Document 1).

Such a process for improving operability in later steps by granulation as in Patent Document 1 shows a certain effect. However, tablets may agglomerate together again during long storage and therefore the agglomerates need to be granulated in a manufacturing process after pulverizing lumps. Therefore, the process of producing adamantane in a large amount becomes inevitably complicated, disadvantageously preventing efficient production.
For manufacturers and fabricators of adamantane, it goes without saying that marketing of adamantane in the form having such a problem is undesirable, and it is a great problem for manufacturers and fabricators to provide adamantane having stable quality.

[Patent Document 1] Japanese Unexamined Patent Publication No.2003-160517

### Disclosure of the Invention

As discussed in Background Art, particles of adamantane agglomerate together, grow into bigger agglomerations over time, and cake inside drums or paper bags for storage. Therefore, the workability in handling adamantane is very low.
An object of the present invention is to provide adamantane having stable quality in which particles are prevented from agglomerating together in such a manner or caking.

The inventors of the present invention conducted extensive research on methods of preventing adamantane from caking to solve the above problem. As a result, the inventors found that controlling the amount of a remaining solvent contained in a produced adamantane to less than a certain level in a process of manufacturing adamantane, in particular controlling the amount of a remaining organic solvent used in purification of adamantane to less than a certain level in a short period of time, can provide adamantane without agglomerations and caking. The inventors also found that in produced adamantane purified by using a naphthene-based solvent, agglomeration and caking are less likely to occur even if the amount of a remaining solvent is greater than in adamantane which is purified by using other solvents. Based on these findings the present invention was achieved. The present invention overturns the common knowledge among persons of skill in the art that adamantane agglomerates.

Thus, the present invention provides the following methods of preventing adamantane from caking:
(1) A method of preventing adamantane from caking, comprising controlling the amount of a solvent contained in adamantane purified by using a naphthene-based solvent to 0.35% by mass or less.
(2) A method of preventing adamantane from caking as defined in (1), wherein the boiling point of a solvent contained in adamantane under atmospheric pressure is 150°C or lower, and the amount of a solvent contained in adamantane is controlled to 0.3% by mass or less by drying for up to 60 minutes.
(3) A method of preventing adamantane from caking as defined in (2), wherein the drying is conducted at a temperature of 50°C or lower and at a pressure of 40 kPa or lower.
(4) A method of preventing adamantane from caking, wherein the amount of a solvent contained in adamantane purified by using a non-naphthene-based solvent is controlled to 0.05% by mass or less.
(5) A method of preventing adamantane from caking as defined in (4), wherein the boiling point of the solvent contained in adamantane at atmospheric pressure is 150°C or lower and the amount of the solvent contained in adamantane is controlled to 0.05% by mass or less by drying for up to 60 minutes.
(6) A method of preventing adamantane from caking as defined in (5), wherein the drying is conducted at a temperature of 50°C or lower and at a pressure of 40 kPa or lower.
(7) A method of preventing adamantane from caking as defined in any one of (1) to (6), wherein adamantane is stored at a temperature of 35°C or lower.

### Brief Description of the Drawings

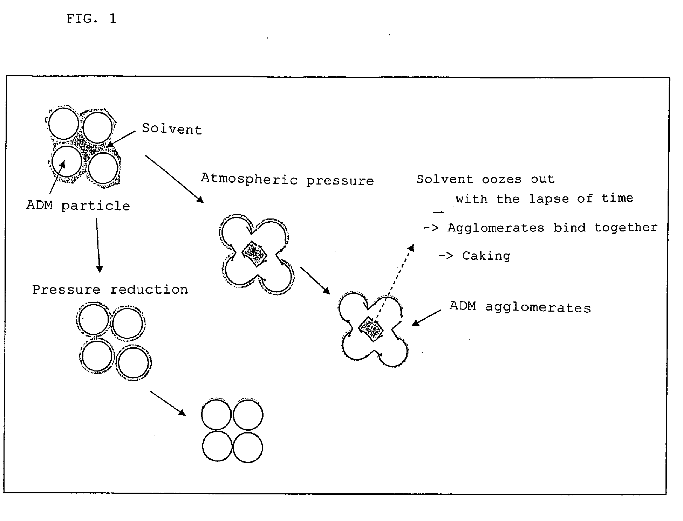

Fig. 1 illustrates the behavior of a solvent in aggregates of adamantane particles (referred to as ADM particles).

### Best Mode for Carrying out the Invention

Adamantane in the present invention is a solid product. Its shape is not particularly limited, but it can be flakes, pulverized or granulated product. Considering the operability in later steps, in general, minute powders having an average grain size of about 100 to 600 µm can be suitably used.
Adamantane is synthesized normally by hydrogenating dicyclopentadiene to obtain trimethylenenorbornane, and isomerizing the obtained trimethylenenorbornane with a catalyst of an acidic substance such as aluminum chloride and hydrogen chloride. However, the reaction yield of the isomerization is not always 100%, endo-trimethylenenorbornane, which is an isomerization raw material, by products such as exo-trimethylenenorbornane and the like remain in a finished product. Therefore, a purification step by washing or occasionally by recrystallization is essential to use it as an industrial raw material. Consequently, a wash solvent or recrystallizing solvent unavoidably remains in a purification product of adamantane. The inventors have found that such remaining solvent further promotes agglomeration of adamantanes which originally sublimes.

The present invention prevents adamantane from caking by controlling in a production process of adamantane the amount of a solvent contained in a produced adamantane to 0.35% by mass or less when purified by using a naphthene-based solvent, or to 0.05% by mass or less when another solvent is used. A solvent remaining inside an adamantane particle hardly evaporates, and the concentration hardly falls after a certain level of a remaining concentration is reached even if the drying time is extended. In other words, if adamantane is left under atmospheric pressure to spontaneously evaporate, the solvent is removed only from the surface area and remains in a high concentration entirely.
Hence, in order to make the amount of the solvent contained in adamantane 0.35% by mass or less or 0.05% by mass or less, the solvent needs to be removed abruptly (for example, under reduced pressure) in a certain short period of time.

That is, in the process of purifying adamantane, a solvent having the boiling point under atmospheric pressure of 150°C or lower is used and the amount of the remaining solvent is reduced to 0.35% by mass or less or 0.05% by mass or less within 60 minutes from the start of the drying step. This allows to produce adamantane which causes no agglomeration in later storage in a natural state and does not cake in normal packages such paper bags even under so-called severe conditions such that pressures are applied to the powders in bags because of the weights of the bags themselves.

As a solvent contained in adamantane, namely, a solvent used for washing or recrystallization for purifying adamantane, those whose boiling point under atmospheric pressure is 150°C or lower are preferred. Since naphthene-based solvents have structures similar to adamantane, part of the remaining solvent is taken into crystals, thereby supposedly preventing caking even if the amount of the remaining solvent is high. Naphthene-based solvents are normally mixtures of C₅ to C₇ hydrocarbons, and consist 50% by mass or more of naphthene-based compounds. Examples of useful non-naphthene-based solvents include, but are not limited to, aliphatic hydrocarbons such as pentanes, hexanes and heptanes, aromatic hydrocarbons such as benzene and toluene, alcohols such as methanol, ethanol, propanol and butanol. Hexane and 2-propanol are suitably used.

The amount of a solvent remaining in a final adamantane product is 0.35% or less, preferably 0.30% by mass or less in case of a naphthene-based solvent, and is 0.05% or less, preferably 0.04% by mass or less in case of a non-naphthene-based solvent. The lowest value of the amount of the remaining solvent is not particularly limited. In terms of drying operation, however, it is normally about 0.05% by mass in case of a naphthene-based solvent, and normally about 0.005% by mass in case of a non-naphthene-based solvent.
That is, in a step using a wash solvent or recrystallizing solvent, which is a purification step in adamantane production, as mentioned above, it is preferable that an organic solvent having the boiling point under atmospheric pressure of 150°C or lower is used; the solvent is removed and dried within 60 minutes from the start of the operation at a temperature of 50°C or lower and under a pressure of 40 kPa or lower to control the amount of a solvent remaining in a final adamantane product to 0.30% by mass or less in case of a naphthene-based solvent. In case of a non-naphthene-based solvent, the amount is preferably 0.04% by mass or less.
A solvent having a boiling point higher than 150°C requires a long drying time even under the above-mentioned pressure or lower. This increases the amount of the solvent remaining inside particles and also promotes sublimation of adamantane itself. Using this solvent is not efficient and makes obtaining adamantane suitable for the present invention difficult.

As mentioned above, a solvent remaining inside an adamantane powder is unlikely to evaporate, and the concentration hardly falls after a certain level of a remaining concentration is reached even if the drying time is extended. However, the remaining solvent can be reduced to a low level by removing the solvent inside the adamantane powder abruptly (for example, under reduced pressure) in a certain short period of time. Stated more specifically, if the adamantane powder is left under atmospheric pressure to spontaneously evaporate, the solvent is removed only from the surface and remains at a high concentration entirely. However, an adamantane powder which hardly cakes can be obtained by causing the solvent to abruptly evaporate under reduced pressure.
Such a characteristic of the adamantane powder will be described with reference to FIG. 1. As shown at the upper left of FIG. 1, adamantane powders are aggregates of adamantane particles (ADM particles), and the adamantane powders after undergoing the purification step are aggregates having the solvent deposited on the surface of its particles.
When this adamantane powder is left to dry under atmospheric pressure (i.e., when it is not vacuum-dried), the surface of the adamantane powder is easily dried, but the solvent within the powders remains. That is, since "dissolution of the surfaces of the particles inside the powders in the solvent" and "deposition of the same from the solvent" are in equilibrium, the particles easily agglomerate together. Once aggregates are formed, the molecules of the solvent which have been remaining on the surface seemingly remain within the powders and are prevented from evaporating. Since the remaining molecules stagnate, the aggregates are coupled to each other more strongly. This is repeated to cause caking.
In contrast, when the adamantane powder after undergoing the purification step is vacuum-dried, the solvent evaporates efficiently from the inside of the adamantane powder at a low temperature. Therefore, since the agglomeration mentioned above is prevented inside the powder and drying is carried out in a state that particles are separated from each other. Therefore, the remaining solvent which tends to stagnate within the aggregates are reduced and the powder is prevented from caking. It can be therefore understood that it is important to efficiently remove the solvent in a short period of time to prevent caking.

Meanwhile, regarding a temperature condition for storing adamantane which has been subjected to the treatment mentioned above, it is important to store at 35°C or lower. Stated more specifically, as long as storage and transportation circumstances at 35°C or lower is maintained, regardless of how long the storage is, no caking in bags or drums is found. In contrast, caking tends to proceed under temperature conditions higher than this. As mentioned above, the phenomenon that caking of adamantane is abruptly stimulated from 35°C has not been known before, and was first found by the inventors of the present invention.
Purified adamantane products may be stored and transported in any manner as long as the temperature condition is maintained at 35°C or lower, and the type of storage and transportation and the shape of containers are not critical. However, to prevent any possible air oxidation during long storage, it is desirable to place under an atmosphere of nitrogen or other inert gas. Moreover, in the present invention, in order to prevent oxidation, adamantane containing an antioxidant can be produced without limitation.

### Example

Subsequently, the present invention will be described in more details with reference to Examples and Comparative Examples, but the present invention is not limited to these Examples.
In each Example and Comparative Example, judgment of the state of adamantane caking (blocking) was carried out by loading a sonic sifting device (Asahi Sonic Sifter A1 made by Kabushiki Kaisha Asahi Seisakusho) with 10 g of adamantane, and determining the amount of adamantane remaining on a 850 µm sift. When the remaining amount is less than 5 g, it was judged not to be caking (O), and when the amount is 5 g or more, it was judged to be caking (X).

### Example 1 (non-naphthene-based solvent)

A twenty-kilogram portion of adamantane particles (0.92% by mass of hydrocarbon having 10 carbon atoms or more) containing 10.00% by mass of 2-propanol (boiling point: 82.4°C) used as purification solvent and having an average particle diameter of 350 µm was dried at a pressure of 10 kPa and a temperature of 50°C for 40 to 60 minutes by using an indirect heat transfer grooved type agitating dryer (capacity: 117L, heat transfer area: 3.88m²).
The amount of 2-propanol contained in the adamantane particles was 0.04% by mass after 40 minutes, and was 0.03% by mass after 60 minutes. The adamantane particles which were subjected to such a treatment were found not caking after being left at 25°C and under atmospheric pressure for 90 days. These adamantane particles were stored under a pressure of 30 kPa by applying a load, but no caking was found after 90 days.

### Example 2

Adamantane particles were dried in a manner similar to Example 1 except that the drying temperature was 40°C. The amount of 2-propanol contained in the adamantane particles was 0.04% by mass after 40 minutes, and was 0.03% by mass after 60 minutes. The adamantane particles which were subjected to such a treatment were found not caking after they were left at 25°C and under atmospheric pressure for 90 days. No caking was found in these adamantane particles which were stored under a pressure of 30 kPa by applying a load for 90 days.

### Example 3

Adamantane particles were dried in a manner similar to Example 1 except that the drying temperature was 0°C. The amount of 2-propanol contained in the adamantane particles was 0.05% by mass after 40 minutes, and was 0.04% by mass after 60 minutes. The adamantane particles which were subjected to such a treatment were found not caking after being left at 25°C and under atmospheric pressure for 90 days. These adamantane particles were found not caking even they were stored under a pressure of 30 kPa by applying a load for 90 days. The samples showed no caking after being stored at 25°C and under atmospheric pressure for 90 days.

### Example 4

Adamantane particles were dried in a manner similar to Example 1 except that the pressure during drying was 40 kPa and the temperature was 40°C. The amount of 2-propanol contained was 0.04% by mass after 40 minutes, and was 0.03% by mass after 60 minutes. The adamantane particles which were subjected to such a treatment were found not caking after being left at 25°C and under atmospheric pressure for 90 days. These adamantane particles were found not caking even after being stored under a pressure of 30 kPa by applying a load for 90 days.

### Example 5

Adamantane particles were dried in a manner similar to Example 2 except that hexane (boiling point: 68.7°C) was used in place of the purification solvent, 2-propanol. The amount of hexane contained was 0.01% by mass after 40 minutes, and was 0.01%. by mass after 60 minutes. The adamantane particles which were subjected to such a treatment were found not caking after being left at 25°C and under atmospheric pressure for 90 days. This sample was found not caking even after being stored at a pressure of 30 kPa by applying a load for 90 days.

### Comparative Example 1

Adamantane particles were dried in a manner similar to Example 4 except that the pressure during drying was changed to 100 kPa. The amount of 2-propanol contained was 0.87% by mass after 40 minutes, and was 0.20% by mass after 60 minutes. Caking was found in the adamantane particles which were subjected to such a treatment after being stored at 2 5°C and under atmospheric pressure for 90 days. Caking was also found in these adamantane particles after being stored under a pressure of 30 kPa by applying a load for 90 days.

### Comparative Example 2

Adamantane particles were dried in a manner similar to Example 5 except that the pressure during drying was changed to 100 kPa. The amount of hexane contained was 0.61% by mass after 40 minutes, and was 0.06% by mass after 60 minutes. Caking was found in the adamantane particles which were subjected to such a treatment after being stored at 25°C and under atmospheric pressure for 90 days. Caking was found in this sample after being stored with a load applied, which was a pressure of 30 kPa.
The operational conditions and evaluation results in the above Examples and Comparative Examples are shown in Table 1.

**Table 1**

| | Example 1 | Example 2 | Example 3 | Example 4 | Example 5 | Comp. Ex-1 | Comp. Ex-2 |
|---|---|---|---|---|---|---|---|
| (Method of producing adamantane) | | | | | | | |
| Solvent in purification step | | | | | | | |
| 2-Propanol | ○ | ○ | ○ | ○ | | ○ | |
| Hexane | | | | | ○ | | ○ |
| Drying step | | | | | | | |
| Pressure (kPa) | 10 | 10 | 10 | 40 | 10 | 100 | 100 |
| Temperature (°C) | 50 | 40 | 0 | 40 | 40 | 40 | 40 |
| (Evalua6on of produced adamantane) | | | | | | | |
| Amount of solvent contained (% by mass) | | | | | | | |
| 40 minutes after start of drying | 0.04 | 0.04 | 0.05 | 0.04 | 0.01 | 0.87 | 0.61 |
| 60 minutes after start of drying | 0.03 | 0.03 | 0.04 | 0.03 | 0.01 | 0.20 | 0.06 |
| Presence or absence of caking | | | | | | | |
| After being stored at room temperature and under atmospheric pressure for 90 days | ○ | ○ | ○ | ○ | ○ | × | × |
| After being stored at room temperature and under a pressure of 30 kPa for 90 days | ○ | ○ | ○ | ○ | ○ | × | × |

### Example 6 (naphthene-based solvent)

The procedure of Example 1 was carried out except that a naphthene-based solvent, Ipzole L (trade name, made by Idemitsu Kosan Co., Ltd.) was used as a wash and purification solvent in Example 1. The operational conditions and evaluation results are shown in Table 2. The compositional outline of Ipzole L is as follows:
methylcyclopentane: 31% by mass
methylpentane: 24% by mass
cyclohexane: 20% by mass and
n-hexane: 15% by mass

### Example 7

Adamantane particles were dried in a manner similar to Example 6 except that the drying temperature was 40°C. The operational conditions and evaluation results are shown in Table 2.

### Comparative Example 3

Adamantane particles were dried in a manner similar to Example 6 except that the drying temperature was 10°C. The operational conditions and evaluation results are shown in Table 2.

### Comparative Example 4

Adamantane particles were dried in a manner similar to Example 7 except that the pressure during drying was 100 kPa. The operational conditions and evaluation results are shown in Table 2.

**Table 2**

| | Example 6 | Example 7 | Comp. Ex 3 | Comp. Ex 4 |
|---|---|---|---|---|
| (Method of producing adamantane) | | | | |
| Solvent in purification step | | | | |
| lpzole-L | ○ | ○ | ○ | ○ |
| Drying step | | | | |
| Pressure (kPa) | 10 | 10 | 10 | 100 |
| Temperature (°C) | 50 | 40 | 10 | 40 |
| (Evaluation of produced adamantane) | | | | |
| Amount of solvent contained (% by mass) | | | | |
| 30 minutes after start of drying | 0.33 | 0.46 | 0.61 | 1.36 |
| 60 minutes after start of drying | 0.20 | 0.27 | 0.38 | 0.70 |
| Presence or absence of caking | | | | |
| After being stored at room temperature and under atmospheric pressure for 90 days | ○ | ○ | Δ | × |
| After being stored at room temperature and under a pressure of 30 kPa for 90 days | ○ | ○ | × | × |

### Example 8 (storage test under atmospheric pressure)

The purified adamantane particles produced under the conditions of Example 2 (the amount of 2-propanol contained 0.04% by mass, average particle diameter: 350 µm) were stored under atmospheric pressure and at a temperature of 0°C, 20°C, 35°C, 38°C, 40°C and 50°C, respectively. After being subjected to a drying treatment, the adamantane particles were judged for their caking state on day 1, 2, 4, 8, 16, 64, 128 and 365. The results are shown in Table 3. It was found that no caking occurred by day 16 at a temperature of 40°C, and at 38°C or lower, no caking occurred even after one-year (365-day) storage under atmospheric pressure (ordinary pressure).

**Table 3**

| Temp. (°C) | Days stored (under atmospheric pressure) | | | | | | | | |
|---|---|---|---|---|---|---|---|---|---|
| | 1 | 2 | 4 | 8 | 16 | 32 | 64 | 128 | 365 |
| 0 | ○ | ○ | ○ | ○ | ○ | ○ | ○ | ○ | ○ |
| 20 | ○ | ○ | ○ | ○ | ○ | ○ | ○ | ○ | ○ |
| 35 | ○ | ○ | ○ | ○ | ○ | ○ | ○ | ○ | ○ |
| 38 | ○ | ○ | ○ | ○ | ○ | ○ | ○ | ○ | ○ |
| 40 | ○ | ○ | ○ | ○ | ○ | × | × | × | × |
| 50 | × | × | × | × | × | × | × | × | × |

### Example 9 (storage test under pressure)

A storage test was conducted in a manner similar to Example 8 except that it was carried out under a pressure of 30 kPa. The results are shown in Table 4. It was found that at 35°C or lower, no caking was observed after one year (365 days) even under a pressurized condition of 30 kPa, while at 38°C or higher, caking already occurred after 2 days; and at 40°C, considerable caking occurred from the first day. Under a more severe temperature condition, 50°C, even worse caking occurred.
Comprehensively, it is considered that a temperature of 35°C or lower enables safe storage.

**Table 4**

| Temp. (°C) | Days stored (under a pressure of 30 kPa) | | | | | | | | |
|---|---|---|---|---|---|---|---|---|---|
| | 1 | 2 | 4 | 8 | 16 | 32 | 64 | 128 | 365 |
| 0 | ○ | ○ | ○ | ○ | ○ | ○ | ○ | ○ | ○ |
| 20 | ○ | ○ | ○ | ○ | ○ | ○ | ○ | ○ | ○ |
| 35 | ○ | ○ | ○ | ○ | ○ | ○ | ○ | ○ | ○ |
| 38 | ○ | × | × | × | × | × | × | × | × |
| 40 | × | × | × | × | × | × | × | × | × |
| 50 | × | × | × | × | × | × | × | × | × |

### Example 10 (naphthene-based solvent: storage test under atmospheric pressure)

A predetermined amount of Ipzole L was added to dried adamantane particles (the amount of remaining Ipzole L: 0.10% by mass) by using Ipzole L as a purification solvent, and the mixture was maintained in a sealed state for 3 weeks at room temperature. The state of blocking after being maintained was determined. The results are shown in Table 5. In Table 5, "IP-L addition" is the amount of Ipzole L added to the adamantane particles as a liquid (% by mass). The "amount of remaining IP-L" is the total amount of the added Ipzole L and the Ipzole L taken in by the starting adamantane particles (% by mass). It can be determined from Table 5 that in case of the naphthene-based solvent (Ipzole L), adamantane can be stored safely without blocking when the amount of the solvent contained in adamantane is less than 0.35% by mass.

**Table 5**

| Sample (adamantane) | IP-L addition (% by mass) | Amount of remaining IP-L (% by mass) | Presence or absence of caking (blocking) |
|---|---|---|---|
| Blank | 0.00 | 0.10 | ○ |
| A | 0.12 | 0.22 | ○ |
| B | 0.23 | 0.33 | ○ |
| C | 0.51 | 0.61 | Δ |
| D | 0.97 | 1.07 | × |
| E | 4.99 | 5.09 | × |

### Industrial Applicability

According to the present invention, the amount of a solvent contained in a produced adamantane in a process of manufacturing of adamantane is controlled to a certain level, whereby caking of adamantane can be prevented and the quality of the product and form of preparation can be stabilized. In addition, pulverization operation before using adamantane, on which considerable efforts have been conventionally spent by customers, is made unnecessary, enabling labor saving.

## Claims

1. A method of preventing adamantane from caking, comprising controlling the amount of a solvent contained in adamantane purified by using a naphthene-based solvent to 0.35% by mass or less.

2. A method of preventing adamantane from caking as defined in claim 1, wherein the boiling point of the solvent contained in adamantane at atmospheric pressure is 150°C or lower and the amount of the solvent contained in adamantane is controlled to 0.3% by mass or less by drying for up to 60 minutes.

3. A method of preventing adamantane from caking as defined in claim 2, wherein the drying is conducted at a temperature of 50°C or lower and at a pressure of 40 kPa or lower.

4. A method of preventing adamantane from caking, wherein the amount of a solvent contained in adamantane purified by using a non-naphthene-based solvent is controlled to 0.05% by mass or less.

5. A method of preventing adamantane from caking as defined in claim 4, wherein the boiling point of the solvent contained in adamantane at atmospheric pressure is 150°C or lower and the amount of the solvent contained in adamantane is controlled to 0.05% by mass or less by drying for up to 60 minutes.

6. A method of preventing adamantane from caking as defined in claim 5, wherein the drying is conducted at a temperature of 50°C or lower and under a pressure of 40 kPa or lower.

7. A method of preventing adamantane from caking as defined in any one of claims 1 to 6, wherein adamantane is stored at a temperature of 35°C or lower.
